(19) Europäisches Patentamt
European Patent Office
Office européen des brevets

(11) Veröffentlichungsnummer: **0 079 312**
**A2**

(12) # EUROPÄISCHE PATENTANMELDUNG

(21) Anmeldenummer: 82810471.1

(22) Anmeldetag: 05.11.82

(51) Int. Cl.³: C 07 D 213/64
C 07 D 213/65, C 07 D 213/70
C 07 D 237/14, C 07 D 239/34
C 07 D 409/06, A 01 N 43/40
A 01 N 43/54, A 01 N 43/58

(30) Priorität: 11.11.81 CH 7257/81

(43) Veröffentlichungstag der Anmeldung:
18.05.83 Patentblatt 83/20

(84) Benannte Vertragsstaaten:
BE CH DE FR GB IT LI NL

(71) Anmelder: CIBA-GEIGY AG
Patentabteilung Postfach
CH-4002 Basel(CH)

(72) Erfinder: Brunner, Hans-Georg, Dr.
Wannenstrasse 14
CH-4415 Lausen(CH)

(72) Erfinder: Schurter, Rolf, Dr.
Holzmattstrasse 45
CH-4102 Binningen(CH)

(72) Erfinder: Szczepanski, Henry, Dr.
Bodenmatt
CH-4323 Wallbach(CH)

(54) **Heterocyclische Acetylenverbindungen.**

(57) Die neuen Verbindungen der Formel I

$$Z-C\equiv C-Het-Y-Q-A \qquad (I)$$

sind bei post-emergenter Anwendung nützliche selektive Herbizide in Getreide, Reis und Mais.

In der Formel I bedeuten

Het einen gegebenenfalls durch ein bis drei Reste R substituierten 5-6-gliedrigen heterocyclischen Rest, der als Ringglieder 1 bis 3 Heteroatome aus der Gruppe Stickstoff, Sauerstoff und Schwefel enthält, wobei Sauerstoff und Schwefel höchstens einmal pro Heterocyclus vorhanden sind,

A eine Gruppe $-NR_1R_2$ oder $-N^{\ominus}HR_1R_2X^{\ominus}$,

$R_1$ und $R_2$ unabhängig voneinander jeweils Wasserstoff, $C_3$-$C_6$-Alkenyl, $C_3$-$C_6$-Alkinyl, $C_3$-$C_8$-Cycloalkyl oder gegebenenfalls durch Halogen, Hydroxy, $C_1$-$C_4$-Alkoxy, Alkoxycarbonyl mit höchstens 5 Kohlenstoffatomen, Cyan oder Carboxyl substituiertes $C_1$-$C_6$-Alkyl, oder

$R_1$ und $R_2$ zusammen mit dem sie tragenden Stickstoffatom auch einen 5- bis 6-gliedrigen gesättigten Heterocyclus mit insgesamt höchstens 2 Heteroatomen, der durch $C_1$-$C_3$-Alkyl substituiert sein kann,

$X^{\ominus}$ ein Anion,

Y Sauerstoff, Schwefel oder ein Radikal $-NR_3-$,

$R_3$ Wasserstoff, $C_3$-$C_6$-Alkenyl, $C_3$-$C_6$-Alkinyl, $C_3$-$C_8$-Cycloalkyl oder gegebenenfalls durch Hydroxy, $C_1$-$C_4$-Alkoxy, Alkoxycarbonyl mit höchstens 5 Kohlenstoffatomen, Cyan oder Carboxyl substituiertes $C_1$-$C_6$-Alkyl,

Q eine unverzweigte oder verzweige $C_2$-$C_6$-Alkylenbrücke,

R Wasserstoff, Nitro, Cyan, Trifluormethyl, $C_1$-$C_4$-Alkoxy, $C_1$-$C_4$-Alkylthio, $-NR_4R_5$, $-CO-NR_6R_7$, $-COOR_8$, $-CO-SR_9$, Halogen, $-N_3$ oder gegebenenfalls durch $C_1$-$C_4$-Alkoxy, Hydroxy, Cyan oder $-COOR_8$ substituiertes $C_1$-$C_4$-Alkyl,

$R_4$, $R_6$, $R_7$, $R_8$ und $R_9$ unabhängig voneinander jeweils Wasserstoff oder $C_1$-$C_6$-Alkyl, $C_3$-$C_8$-Alkenyl oder $C_3$-$C_8$-Alkinyl,

$R_5$ Wasserstoff, $C_1$-$C_6$-Alkyl, $C_3$-$C_8$-Alkenyl, $C_3$-$C_8$-Alkinyl, $-CO-R_{10}$, $-COO-R_{11}$ oder $-CO-NHR_{12}$ darstellen, wobei $R_{10}$, $R_{11}$ und $R_{12}$ die gleiche Bedeutung wie $R_3$ haben und

Z für einen gegebenenfalls durch ein bis drei Reste, die dieselbe Bedeutung haben wie für R angegeben, oder Formyl, $-SO_2-NR_6R_7$, $-NH-NH_2$, $-NHOH$, $-SO-R_8$, $-SO_2-R_8$,

$-N=CH-NR_6R_7$, $COO^{\ominus}$ $M^{\oplus}$ oder gegebenenfalls durch Nitro, Cyano oder $-COOR_8$ substituiertes $C_2$-$C_6$-Alkenyl bedeuten, substituierten Phenyl-, Naphthyl- oder heterocyclischen, aromatischen Rest steht, der mindestens ein Sauerstoff-, Schwefel- oder Stickstoffatom enthält, wobei $M^{\oplus}$ für ein Natrium-, Kalium-, Calcium- oder Magnesium-Kation steht.

EP 0 079 312 A2

CIBA-GEIGY AG
Basel (Schweiz)

5-13654/=

# Heterocyclische Acetylenverbindungen

Die vorliegende Erfindung betrifft neue heterocyclische Acetylenverbindungen mit herbizider Wirkung, Verfahren zu deren Herstellung, sie enthaltende Mittel, sowie deren Verwendung zur selektiven Unkrautbekämpfung in verschiedenen Nutzpflanzenkulturen, wie z.B. Getreide, Mais und Reis. Die Erfindung betrifft ausserdem als Zwischenprodukte dienende neue 1,1-Dialkyl-3-heterocyclo-propargylalkohole und deren Herstellung.

Die erfindungsgemässen neuen heterocyclischen Acetylenverbindungen entsprechen der allgemeinen Formel I

$$Z - C \equiv C - Het - Y - Q - A \qquad (I),$$

worin

Het einen gegebenenfalls durch ein bis drei Reste R substituierten 5-6-gliedrigen heterocyclischen Rest, der als Ringglieder 1 bis 3 Heteroatome aus der Gruppe Stickstoff, Sauerstoff und Schwefel enthält, wobei Sauerstoff und Schwefel höchstens einmal pro Heterocyclus vorhanden sind,

A eine Gruppe $-NR_1R_2$ oder $-\overset{\oplus}{N}HR_1R_2X^{\ominus}$,

$R_1$ und $R_2$ unabhängig voneinander jeweils Wasserstoff, $C_3$-$C_6$-Alkenyl, $C_3$-$C_6$-Alkinyl, $C_3$-$C_8$-Cycloalkyl oder gegebenenfalls durch Halogen, Hydroxy, $C_1$-$C_4$-Alkoxy, Alkoxycarbonyl mit höchstens 5 Kohlenstoffatomen, Cyan oder Carboxyl substituiertes $C_1$-$C_6$-Alkyl, oder

$R_1$ und $R_2$ zusammen mit dem sie tragenden Stickstoffatom auch einen 5- bis 6-gliedrigen gesättigten Heterocyclus mit insgesamt höchstens 2 Heteroatomen, der durch $C_1$-$C_3$-Alkyl substituiert sein kann,

$X^{\ominus}$ ein Anion,

Y Sauerstoff, Schwefel oder ein Radikal $-NR_3-$,

$R_3$ Wasserstoff, $C_3$-$C_6$-Alkenyl, $C_3$-$C_6$-Alkinyl, $C_3$-$C_8$-Cycloalkyl oder gegebenenfalls durch Hydroxy, $C_1$-$C_4$-Alkoxy, Alkoxycarbonyl mit höchstens 5 Kohlenstoffatomen, Cyan oder Carboxyl substituiertes $C_1$-$C_6$-Alkyl,

Q eine unverzweigte oder verzweigte $C_2$-$C_6$-Alkylenbrücke,

R Wasserstoff, Nitro, Cyan, Trifluormethyl, $C_1$-$C_4$-Alkoxy, $C_1$-$C_4$-Alkylthio, $-NR_4R_5$, $-CO-NR_6R_7$, $-COOR_8$, $-CO-SR_9$, Halogen, $-N_3$ oder gegebenenfalls durch $C_1$-$C_4$-Alkoxy, Hydroxy, Cyan oder $-COOR_8$ substituiertes $C_1$-$C_4$-Alkyl,

$R_4$, $R_6$, $R_7$, $R_8$ und $R_9$ unabhängig voneinander jeweils Wasserstoff oder $C_1$-$C_6$-Alkyl, $C_3$-$C_8$-Alkenyl oder $C_3$-$C_8$-Alkinyl,

$R_5$ Wasserstoff, $C_1$-$C_6$-Alkyl, $C_3$-$C_8$-Alkenyl, $C_3$-$C_8$-Alkinyl, $-CO-R_{10}$, $-COO-R_{11}$ oder $-CO-NHR_{12}$ darstellen, wobei

$R_{10}$, $R_{11}$ und $R_{12}$ die gleiche Bedeutung wie $R_3$ haben und

Z für einen gegebenenfalls durch ein bis drei Reste, die dieselbe Bedeutung haben wie für R angegeben, oder Formyl, $-SO_2NR_6R_7$, $-NH-NH_2$, $-NHOH$, $-SO-R_8$, $-SO_2-R_8$, $-N=CH-NR_6R_7$, $COO^{\ominus}M^{\oplus}$ oder gegebenenfalls durch Nitro, Cyano oder $-COOR_8$ substituiertes $C_2$-$C_6$-Alkenyl bedeuten, substituierten Phenyl-, Naphthyl- oder heterocyclischen, aromatischen Rest steht, der mindestens ein Sauerstoff-, Schwefel- oder Stickstoffatom enthält, wobei $M^{\oplus}$ für ein Natrium-, Kalium-, Calcium- oder Magnesium-Kation steht.

Herbizid wirksame Diarylacetylenverbindungen sind erst vor kurzem in der europäischen Patentanmeldung Nr. 81810195.8 und in Phytochemistry 19, 61 (1980) beschrieben worden.

In der Definition der erfindungsgemässen Verbindungen der Formel I steht Het für einen 5-6-gliedrigen Heterocyclus, der aromatischen oder nichtaromatischen Charakter haben kann.

Beispiele für aromatische Heterocyclen, die für die Symbole Het oder Z stehen können, sind Furan, Thiophen, Triazol, Pyridin, Oxazol, Thiazol, Thiadiazol, Pyrrol, Imidazol, Furazan, Pyrazol, Pyrazin, Pyrimidin, Pyridazin, symmetrisches und asymmetrisches Triazin oder Oxadiazol, vorzugsweise jedoch Furan, Thiophen, Pyridin, Pyrimidin, Pyrazin, Pyridazin, Thiazol, Thiadiazol, Oxadiazol, Oxazol, Pyrazol, Triazol, Imidazol und Triazin.

Beispiele für gesättigte Heterocyclen, die von $R_1$ und $R_2$ unter Einschluss des sie bindenden Stickstoffatoms gebildet werden können, sind Pyrrolidin, Imidazolidin, Pyrazolidin, Piperidin, Piperazin, Piperimidin, Morpholin und Thiomorpholin.

Beispiele für Alkyl in den Definitionen von R und $R_1$ bis $R_{12}$ sind Methyl, Aethyl, n-Propyl, i-Propyl, die vier isomeren Butyl sowie die geradkettigen oder verzweigten Pentyl- oder Hexylreste, vorzugsweise ist der Alkylrest jedoch gerad- und kurzkettig, insbesondere ist er durch Aethyl oder Methyl verkörpert.

Beispiele für Alkoxy in den Substituenten-Definitionen sind Methoxy, Aethoxy, n-Propyloxy, i-Propyloxy oder die vier isomeren Butyloxyreste, vorzugsweise jedoch Methoxy oder Aethoxy.

Beispiele für Alkylthio sind Methylthio, Aethylthio, n-Propylthio oder n-Butylthio, vorzugsweise jedoch Methylthio oder Aethylthio.

Beispiele für $C_3$-$C_8$-Cycloalkyl sind Cyclopropyl, Cyclobutyl, Cyclopentyl, Cyclohexyl, Cycloheptyl oder Cyclooctyl, vorzugsweise jedoch Cyclopropyl oder vor allem Cyclopentyl oder Cyclohexyl.

Beispiele für Anionen $X^{\ominus}$ sind die Anionen organischer und anorganischer Säuren wie Halogenwasserstoffsäuren, Phosphorsäure, Schwefelsäure, aliphatischer und aromatischer Sulfonsäuren, Fettsäuren sowie mehrwertiger, organischer Säuren wie Oxalsäure, Malonsäure, Bernsteinsäure, Adipinsäure und Zitronensäure, vorzugsweise sind die Anionen jedoch Halogenidanionen, wie Chlorid oder Bromid.

Beispiele für Alkenylreste sind Allyl, die isomeren Butenyl-, Pentenyl-, Hexenyl-, Heptenyl- und Octenylreste, insbesondere aber Allyl.

Beispiele für Alkinylreste sind Propargyl, die isomeren Butinyl-, Pentinyl-, Hexinyl-, Heptinyl- und Octinylreste, insbesondere aber Propargyl.

Bevorzugte Halogensubstituenten sind Chlor, Brom und Jod.

Die Alkylenbrücke Q wird durch geradkettige oder verzweigte Strukturen verkörpert, wie z.B.: 1,2-Aethylen; 1,3-Propylen; 1,4-Butylen; 1,5-Pentylen; 1-Methyl-1,2-äthylen; 2-Methyl-1,2-äthylen; 1-Aethyl-1,2-äthylen; 2-Aethyl-1,2-äthylen; 1,2-Dimethyl-1,2-äthylen; 1-Methyl-1,3-propylen; 2-Methyl-1,3-propylen; 3-Methyl-1,3-propylen; 1-Aethyl-1,3-propylen; 2-Methyl-1,3-propylen; vorzugsweise besteht die direkte Kette zwischen dem Brückenatom Y und dem Stickstoffatom jedoch aus 2 oder 3 Kohlenstoffatomen.

Von den Verbindungen der Formel I sind diejenigen bevorzugt, in denen

a) der Heterocyclus Het einen gegebenenfalls substituierten aromatischen 6-gliedrigen Stickstoffring wie Pyridin, Pyrazin, Pyridazin, Pyrimidin oder Triazin,
b) der Heterocyclus Het einen gegebenenfalls substituierten aromatischen 5-Ring wie Furan, Thiophen, Pyrazol, Imidazol, Triazol, Oxazol, Thiazol, Thiadiazol oder Oxadiazol,
c) Z gegebenenfalls substituiertes Phenyl oder Thienyl bedeutet,

d) die direkte Alkylenbrücke zwischen dem Brückenatom Y und dem Stickstoffatom der Gruppe A aus 2 oder 3 Kohlenstoffatomen besteht,
e) die Reste $R_1$ und $R_2$ für Methyl oder Aethyl stehen, und
f) das Brückenglied Y Sauerstoff ist.

Durch Kombination einzelner Merkmale der Gruppen a-f ergeben sich die weiter bevorzugten Untergruppen von Verbindungen der Formel I, in denen

aa) Het einen gegebenenfalls substituierten Pyridin-, Pyrazin-, Pyridazin-, Pyrimidin- oder Triazinring, Z einen gegebenenfalls substituierten Phenyl- oder Thienylring, $R_1$ und $R_2$ Methyl oder Aethyl und Y Sauerstoff bedeuten und die direkte Alkylenbrücke zwischen dem Brückenatom Y und dem Stickstoffatom der Gruppe A aus 2 oder 3 Kohlenstoffatomen besteht und
bb) Het einen gegebenenfalls substituierten Furan-, Thiophen-, Imidazol-, Triazol-, Oxazol-, Thiazol-, Thiadiazol- oder Oxadiazolring, Z einen gegebenenfalls substituierten Phenyl- oder Thienylring, $R_1$ und $R_2$ Methyl oder Aethyl und Y Sauerstoff bedeuten und die direkte Alkylenbrücke zwischen dem Brückenatom Y und dem Stickstoffatom der Gruppe A aus 2 oder 3 Kohlenstoffatomen besteht.

Innerhalb der Gruppe aa sind wiederum die Verbindungen bevorzugt, in denen

ab) Het einen gegebenenfalls substituierten Pyridin- oder Pyrimidinring und Z einen gegebenenfalls substituierten Thiophenring und
ac) Het einen gegebenenfalls substituierten Pyridin- oder Pyrimidinring und Z einen gegebenenfalls substituierten Phenylring bedeuten.

Einen ganz besonderen Vorzug geniessen unter den Verbindungen der letztgenannten Untergruppen ab) und ac) diejenigen Verbindungen, in denen die Alkylenkette zwischen dem Brückenatom Y und dem Stickstoffatom der Gruppe A 2 Kohlenstoffatome enthält.

Als bevorzugte Einzelverbindungen sind zu nennen:
1-(5-Phenyläthinyl-pyridyl-2-oxy)-2-diäthylaminoäthan,
2-(5-Phenyläthinyl-pyridyl-2-oxy)-1-diäthylaminopropan und
1-[5-(2-Thienyläthinyl)-pyridyl-2-oxy)-2-diäthylaminoäthan.

Die erfindungsgemässen Verbindungen der Formel I können entsprechend dem folgenden Schema A hergestellt werden. Die Formeln IV und X sind dabei als Unterformeln der Formel I zu verstehen.

Scheme A:

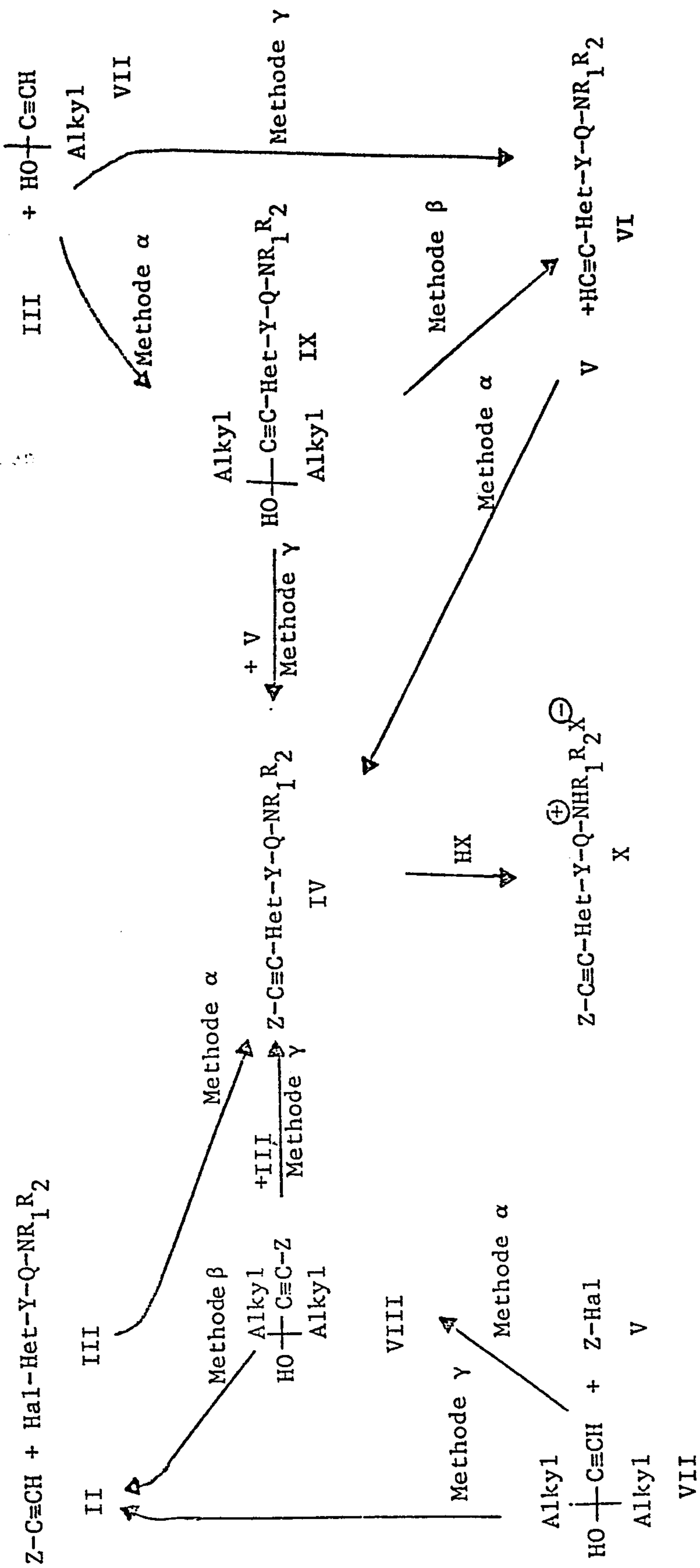
Methode α
Methode β
Methode γ
+ V
+III
HX
II
III
IV
V
VI
VII
VIII
IX
X

Im Schema A haben Het, $R_1$, $R_2$, Q, X, Y und Z die unter Formel I angegebene Bedeutung. Unter Hal ist Chlor, insbesondere aber Brom und Jod zu verstehen.

Methode α ist ein Verfahren, das es erlaubt, unter Verwendung von Metallkatalysatoren, halogenierte aromatische Reste, wie in den Formeln III oder V, mit endständigen Acetylengruppen, wie in den Formeln II, VI und VII, unter milden Reaktionsbedingungen in Gegenwart eines säurebindenden Mittels zu binden. Reaktionen dieser Art sind in folgenden Literaturstellen beschrieben: K. Sonogashira, Y. Tohda und N. Hagihara, Tetrahedron Lett., 50, 4467 (1975):, L. Cassar, J. Organomet. Chem., 93, 253 (1975) und H.A. Dieck und F.R. Heck, J.Organomet. Chem., 93, 259 (1975).

Diese Reaktion findet vorteilhafterweise in gegenüber den Reaktionspartnern inerten, organischen Lösungsmitteln statt. Als solche kommen viele protische wie auch aprotische Lösungsmittel in Frage, z.B. Alkohole, Ketone, Aether, Kohlenwasserstoffe, halogenierte Kohlenwasserstoffe, aromatische Lösungsmittel: wie Methanol, Aethanol, Isopropanol, Cyclohexanon, Aceton, Methyläthylketon, Diäthyläther, Dimethyläther, Tetrahydrofuran, Dioxan, Cyclohexan, Pentan, Hexan, Heptan, Octan, Methylenchlorid, Chloroform, Tetrachlorkohlenstoff, Benzol, Toluol oder Xylol oder auch z.B. Dimethylformamid, Dimethylsulfoxid, Acetonitril oder tertiäre Amine wie Triäthylamin.

Als säurebindendes Mittel kann, da bei der Umsetzung Halogenwasserstoff abgespalten wird, eine Base eingesetzt werden. Dafür kommen starke anorganische Basen wie KOH, NaOH in Frage, aber auch organische wie beispielsweise Triäthylamin, Diäthylamin, Pyridin, Alkoholate etc. Bei den Reaktionen werden gegebenenfalls 1-5 Aequivalente dieser Basen eingesetzt.

Als Metallkatalysatoren werden vorzugsweise Palladiumsalze oder -komplexe verwendet, insbesondere Palladiumchlorid $PdCl_2$, Palladiumacetat $Pd(OCOCH_3)_2$ oder der Palladiumdichlor-bis-(triphenylphosphin)-Komplex $Pd\ Cl_2[P(C_6H_5)_3]_2$, meist unter Zusatz eines Kupfer-(I)-Salzes, insbesondere von Kupfer-(I)-jodid. Die Katalysatoren werden als solche oder aufgezogen auf einem Träger wie z.B. Kohlepulver, Aluminiumoxid etc., verwendet.

Die Reaktionstemperaturen liegen in der Regel zwischen 0° und 200°C, vorwiegend jedoch zwischen Raumtemperatur und der Siedetemperatur des Reaktionsgemisches.

Die Reaktionszeiten liegen im allgemeinen zwischen 0,5 und 48 Stunden.

Die Methode β ermöglicht es, in Gegenwart einer starken Base wie NaOH,KOH oder Alkoholat aus einem tertiären Aethinylalkohol, wie in den Formeln VII, VIII und IX, welches als geschützte endständige Acetylengruppe aufgefasst werden kann, unter Abspaltung der Ketoschutzgruppe das Acetylen, wie in den Formeln II, VI und IX, freizusetzen. Die als Nebenprodukte entstehenden Ketone können aus dem Reaktionsgemisch während der Reaktion destillativ entfernt werden. Reaktionen dieser Art sind in der DOS 2 905 507 und im US-Patent 4 128 588 beschrieben.

Diese Reaktion wird vorteilhafterweise in gegenüber den Reaktionspartnern inerten, organischen Lösungsmitteln wie Alkoholen, Aethern, Ketonen, Kohlenwasserstoffen, Halogenkohlenwasserstoffen, aromatischen Lösungsmitteln oder auch Dimethylformamid, Dimethylsulfoxid oder Acetonitril durchgeführt. Beispiele für solche Lösungsmittel sind: Methanol, Aethanol, Isopropanol, Dimethyläther, Diäthyläther, Tetrahydrofuran, Dioxan, Aceton, Methyläthylketon, Cyclohexanon, Cyclohexan, Benzol, Toluol, Xylol, Methylenchlorid, Chloroform oder Tetrachlorkohlenstoff.

Die Reaktionstemperatur liegt auch hier vorzugsweise zwischen Raumtemperatur und dem Siedepunkt des Reaktionsgemisches.

Die Reaktionszeit beträgt im allgemeinen zwischen 0,5 und 12 Stunden.

Die Methode γ besteht aus einer Kombination der Methoden α und β, wobei das nach der Methode α umzusetzende Acetylen in situ durch Einwirkung einer starken Base auf ein geschütztes Acetylen der Formeln VII, VIII und IX hergestellt wird.

Die Reaktionsbedingungen sind mit denen der Methode α identisch.

Zwingend erforderlich ist jedoch der Zusatz einer starken Base wie NaOH, KOH oder das Alkalisalz eines Alkohols.

Nach dem erfindungsgemässen Verfahren werden die Verbindungen der Formel I erhalten, indem man entweder eine Aethinylverbindung der Formel II

$$Z - C \equiv CH \qquad (II)$$

in Gegenwart eines säurebindenden Mittels und eines Metallkatalysators gegebenenfalls unter einer Inertgasatmosphäre mit einem heterocyclischen Halogenid der Formel III

$$Hal - Het - Y - Q - NR_1R_2 \qquad (III)$$

umsetzt und gegebenenfalls in Ammoniumsalze überführt oder indem man ein aromatisches Halogenid der Formel V

$$Z - Hal \qquad (V)$$

unter den gleichen Reaktionsbedingungen mit einem heterocyclischen Acetylen-Derivat der Formel VI

$$HC \equiv C - Het - Y - Q - NR_1R_2 \qquad (VI),$$

worin Het, $R_1$, $R_2$, Y, Q und Z die unter Formel I gegebene Bedeutung haben und Hal Brom oder Jod ist, umsetzt und gegebenenfalls in die Ammoniumsalze überführt.

Nach einem weiteren erfindungsgemässen Verfahren werden die Verbindungen der Formel I erhalten, indem man ein aromatisches Halogenid der Formel V

$$Z - Hal \qquad (V)$$

in Gegenwart eines säurebindenden Mittels und eines Metallkatalysators gegebenenfalls unter einer Inergasatmosphäre mit einem Propargyl-. alkohol der Formel VII

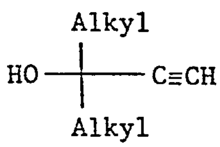

(VII)

umsetzt und die erhaltene Aethinylverbindung der Formel VIII

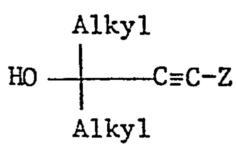

(VIII)

in Gegenwart einer starken Base und eines Metallkatalysators gegebenenfalls unter einer Inertgasatmosphäre mit einem heterocyclischen Halogenid der Formel III

$$Hal - Het - Y - Q - NR_1R_2 \qquad (III)$$

umsetzt und gegebenenfalls in die Ammoniumsalze überführt oder indem man das heterocyclische Halogenid der Formel III unter den obigen Bedingungen zuerst mit dem Propargylalkohol der Formel VII umsetzt und dann die entstandene Aethinylverbindung der Formel IX

$$HO-\overset{Alkyl}{\underset{Alkyl}{C}}-C{\equiv}C\text{-Het-Y-Q-}NR_1R_2 \qquad (IX)$$

unter den obigen Bedingungen mit dem aromatischen Halogenid der Formel V, worin Het, $R_1$, $R_2$, Q und Z die unter Formel IV gegebene Bedeutung haben, Hal Brom oder Jod ist und Alkyl ein $C_1$-$C_4$-Alkylrest ist, umsetzt und gegebenenfalls in die Ammoniumsalze überführt.

Die ebenfalls neuen und speziell als Zwischenprodukte für die Verbindungen der Formel I entwickelten 1,1-Dialkyl-3-heterocyclo-propargylalkohole der Formel IX werden analog zum ersten Reaktionsschritt des vorgenannten Verfahrens hergestellt, indem man Halogenide der Formel III mit einem Propargylalkohol der Formel VII umsetzt.

Die Verbindungen der Formel IX bilden ebenfalls einen Gegenstand der vorliegenden Erfindung.

Nach einem weiteren Verfahren erhält man die erfindungsgemässen Verbindungen der Formel X

$$Z-C{\equiv}C\text{-Het-Y-Q-}\overset{\oplus}{N}HR_1R_2X^{\ominus} \qquad (X),$$

indem man die Verbindungen der Formel IV mit einer Säure HX umsetzt und das Produkt isoliert, worin Het, $R_1$, $R_2$, Q, X, Y und Z die unter Formel I und Ia gegebene Bedeutung haben.

Die Ausgangsmaterialien der Formeln II, III, V, VI und VII sind bekannt oder leicht herstellbar und/oder im Handel erhältlich. Zwischenprodukte der Formel VIII sind in der europäischen Patentanmeldung Nr. 41476 beschrieben.

Die neuen Wirkstoffe der Formel I sind stabile Verbindungen.

Die Verbindungen der Unterformel IV sind in den üblichen organischen

Lösungsmitteln relativ gut und in Wasser schlecht löslich. Sie können durch Zugeben von Wasser zur Reaktionslösung leicht ausgefällt werden. Ihre Formulierung als flüssige herbizide Mittel gelingt mit Hilfe üblicher Lösungsvermittler und/oder Dispergiermittel.

Die Verbindungen der Unterformel X sind in Wasser gut, jedoch selbst in polaren organischen Lösungsmitteln wie Dimethylsulfoxid, Dimethylformamid oder Acetonitril relativ schlecht löslich.

Die erfindungsgemässen heterocyclischen Acetylen-Verbindungen der Formel I beeinflussen das Pflanzenwachstum und zeigen bei post-emergenter Applikation ausgezeichnete selektiv-herbizide Wirkung gegen schwer bekämpfbare breitblättrige Unkräuter wie Galium, Veronica, Viola, aber auch gegen Sinapis, Chrysanthemum etc. in Mais und Reis, vorwiegend aber in Getreide-Kulturen. Besonders mehrjährige Unkräuter werden durch die Translokation der Wirkstoffe der Formel I wirksam bekämpft.

Unter Translokation ist der Transport eines Wirkstoffes innerhalb der Pflanze zu verstehen. Dabei kann der Wirkstoff von den Blättern in die Wurzel und umgekehrt transloziert werden und dort seine Wirkung entfalten. Einige der Wirkstoffe der Formel I sind translozierbar, d.h. mit ihnen werden z.B. durch Blattapplikation Unkräuter bis in die Wurzel hinein geschädigt.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die

Art der Mittel den angestrebten Zielen und den gegebenen Verhätnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidertes Kokusnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Doedecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und

Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979.
Sisley and Wood, "Encyclopedia of Surface Active Agents", Chemical Publishing Co., Inc. New York, 1964.

Die pestiziden Zubereitungen enthalten in der Regel 0,1 bis 99 %, insbesondere 0,1 bis 95%, Wirkstoff der Formel I, 1 bis 99% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

Lösungen

| | |
|---|---|
| Aktiver Wirkstoff: | 5 bis 95%, vorzugsweise 10 bis 80% |
| Lösungsmittel: | 95 bis 5%, vorzugsweise 90 bis 0% |
| oberflächenaktives Mittel: | 1 bis 30%, vorzugsweise 2 bis 20%. |

Emulgierbare Konzentrate:

| | |
|---|---|
| Aktiver Wirkstoff: | 10 bis 50%, bevorzugt 10 bis 40% |
| oberflächenaktives Mittel: | 5 bis 30%, vorzugsweise 10 bis 20% |
| flüssiges Trägermittel: | 20 bis 95%, vorzugsweise 40 bis 80%. |

Stäube

| | |
|---|---|
| Aktiver Wirkstoff: | 0,5 bis 10%, vorzugsweise 2 bis 8% |
| festes Trägermittel: | 99,5 bis 90%, vorzugsweise 98 bis 92%. |

Suspension-Konzentrate

| | |
|---|---|
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |

Benetzbare Pulver

| | |
|---|---|
| Aktiver Wirkstoff: | 5 bis 90%, vorzugsweise 10 bis 80% und insbesondere 20 bis 60% |
| oberflächenaktives Mittel: | 0,5 bis 20%, vorzugsweise 1 bis 15% |
| festes Trägermittel: | 5 bis 90%, vorzugsweise 30 bis 70%. |

Granulate

| | |
|---|---|
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,1 bis 10 kg AS/ha, vorzugsweise 0,25 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Herstellungsbeispiele

Beispiel 1: 1-(5-Phenyläthinyl-pyridyl-2-oxy)-2-diäthylaminoäthan

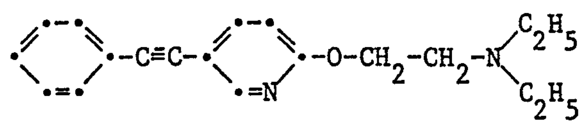

Einer Lösung von 44 g (0,137 Mol) 2-(2-Diäthylaminoäthoxy)-5-jodpyridin und 22 ml (0,192 Mol) Phenylacetylen in 350 ml Triäthylamin werden unter Stickstoff 1 g Palladiumkomplex $PdCl_2[P(C_6H_5)_3]_2$ und 500 mg Kupfer-I-jodid CuJ zugesetzt. Die Reaktionsmischung erwärmt sich auf 65° und wird hochviskos. Nach Rühren der Mischung unter Abkühlen auf Raumtemperatur für 6 Stunden und Eindampfen wird der Rückstand in Aether aufgenommen und viermal mit Eiswasser gewaschen. Durch Abdampfen des Aethers erhält man 35 g (86,5%) 1-(5-Phenyläthinyl-pyridyl-2-oxy)-2-diäthylaminoäthan als Wachs.

Beispiel 2: 2-(5-Phenyläthinyl-pyridyl-2-oxy)-1-diäthylaminopropan

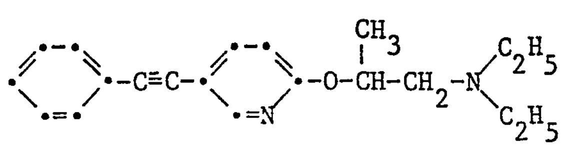

a) Einer Lösung von 48 g (0,19 Mol) 2-Chlor-5-jodpyridin, 20,5 g (0,2 Mol) Phenylacetylen in 350 ml Triäthylamin werden unter Stickstoff 500 mg Kupfer-I-jodid und 1 g Palladiumkomplex $PdCl_2[P(C_6H_5)_3]_2$ zugesetzt. Nach Abklingen der anfangs schwach exothermen Reaktion wird das Reaktionsgemisch bei Raumtemperatur für 18 Stunden gerührt,

eingeengt, und auf 300 ml Eiswasser gegossen. Das ausgefallene Produkt wird abfiltriert, mit Wasser nachgewaschen und getrocknet. Man erhält so 36 g (84%) 2-Chlor-5-phenyläthinyl-pyridin, Smp. 70-72°.

b) Einer Lösung von 15 ml (0,102 Mol) 1-Diäthylamino-2-propanol in 50 ml Xylol setzt man portionsweise 750 mg Natrium zu und erwärmt die Lösung auf 80° bis zur Beendigung der Wasserstoffentwicklung. Danach werden der Reaktionsmischung 6,4 g (0,03 Mol) 2-Chlor-5-phenyläthinyl-pyridin zugefügt und die Mischung für 5 Stunden am Rückfluss gekocht. Das abgekühlte Reaktionsgemisch wird mit 100 ml Aether versetzt, dreimal mit Wasser gewaschen, getrocknet und eingedampft. Durch Entfernen von Lösungsmittelresten im Hochvakuum (2 mb) erhält man 8,9 g (96,5%) 2-(5-Phenyläthinyl-pyridyl-2-oxy)-1-diäthylaminopropan, $n_D^{25} = 1,5368$.

Beispiel 3: 1-[5-(2-Thienyläthinyl)-pyridyl-2-oxy)-2-diäthylaminoäthan

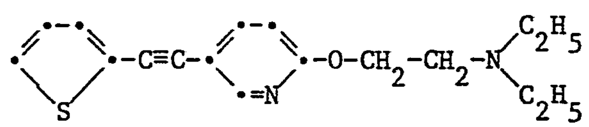

a) Einer Lösung von 28 g (0,0875 Mol) 2-(2-Diäthylamino-äthoxy)-5-jod-pyridin und 8,4 g (0,1 Mol) 3-Methyl-1-butin-3-ol in 200 ml Triäthylamin werden unter Stickstoff 500 mg $PdCl_2[P(C_6H_5)_3]_2$-Komplex und 250 mg Kupfer-I-jodid zugesetzt. Nach Rühren für 18 Stunden bei Raumtemperatur wird die Reaktionsmischung eingeengt, der Rückstand in Aether aufgenommen, dreimal mit Eiswasser gewaschen, getrocknet und eingedampft. Man erhält so 19 g (96%) 2-(2-Diäthylamino-äthoxy)-5-(3-methyl-3-hydroxy-1-butinyl)-pyridin, das ohne weitere Reinigung weiter verwendet wird.

b) Ein Gemisch aus 7,5 g (0,0332 Mol) rohem 2-(2-Diäthylamino-äthoxy)-5-(3-methyl-3-hydroxy-1-butinyl)-pyridin, 6,3 g (0,03 Mol) 2-Jodthiophen, 3 g pulverisiertem Kaliumhydroxid, 300 mg $PdCl_2[P(C_6H_5)_3]_2$-Komplex, 150 mg Kupfer-I-jodid und 100 ml Triäthylamin werden für 18 Stunden zum Rückfluss erhitzt. Nach dem Abkühlen wird das Gemisch

eingeengt, in Aethylacetat aufgenommen, dreimal mit Eiswasser gewaschen, getrocknet und eingedampft. Durch Filtration über eine kurze Silicagelsäule mit Chloroform/Methanol (10:1) erhält man nach Befreiung vom Lösungsmittel 4,4 g (44%) 1-[5-(2-Thienyläthinyl)-pyridyl-2-oxy]-2-diäthylaminoäthan, $n_D^{25}$ : 1,6176.

Die aus den Beispielen erhaltenen und in analoger Weise herzustellenden Verbindungen sind in den folgenden Tabellen aufgeführt.

Tabelle 1:

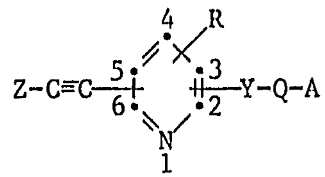

| Nr. | Z - | Stellung von $-C\equiv C-Z$ | - Y-Q-A | Stellung von -Y-Q-A | R | phys.Daten |
|---|---|---|---|---|---|---|
| 1 | $C_6H_5-$ | 5 | $-OCH_2-CH_2-N(C_2H_5)_2$ | 2 | H | Wachs |
| 2 | $C_6H_5-$ | 5. | $-OCH(CH_3)-CH_2-N(C_2H_5)_2$ | 2 | H | $n_D^{25}$ 1.5368 |
| 3 | $C_6H_5-$ | 5 | $-OCH_2-CH(CH_3)-N(C_2H_5)_2$ | 2 | H | $n_D^{25}$ 1.6048 |
| 4 | 2-Thienyl- | 5 | $-OCH_2-CH_2-N(C_2H_5)_2$ | 2 | H | $n_D^{25}$ 1.6176 |
| 5 | $4-F-C_6H_4-$ | 5 | $-OCH_2-CH_2-N(C_2H_5)_2$ | 2 | H | $n_D^{25}$ 1.5852 |
| 6 | $4-OCH_3-C_6H_4-$ | 5 | $-OCH_2-CH_2-N(C_2H_5)_2$ | 2 | H | Harz |
| 7 | $C_6H_5-$ | 2 | $-OCH_2-CH_2-N(C_2H_5)_2$ | 5 | H | Harz |
| 8 | $C_6H_5-$ | 6 | $-OCH_2-CH_2-N(CH_3)C_2H_5$ | 2 | H | |
| 9 | $C_6H_5-$ | 2 | $-OCH_2-CH_2-N(C_3H_7)_2$ | 4 | H | |
| 10 | $4-CN-C_6H_4-$ | 2 | $-OCH_2-CH_2-$ 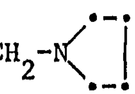 | 4 | H | |
| 11 | $C_6H_5-$ | 5 | $-OCH_2-CH_2-N(CH_3)_2$ | 2 | 3-CN | |
| 12 | $C_6H_5-$ | 5 | $-OCH_2-CH_2-N(CH_3)_2$ | 2 | 3-Cl | |
| 13 | $C_6H_5-$ | 2 | $-OCH_2-CH_2-N(CH_3)_2$ | 5 | 3-Cl | |
| 14 | $C_6H_5-$ | 5 | $-SCH_2-CH_2-N(C_2H_5)_2$ | 2 | H | $n_D^{21}$ 1.6333 |

Tabelle 1 (Fortsetzung)

| Nr. | Z - | Stellung von $-C{\equiv}C-Z$ | -Y-Q-A | Stellung von -Y-Q-Y | R | phys.Daten |
|---|---|---|---|---|---|---|
| 15 | 2-Thienyl- | 5 | $-SCH_2-CH_2-N(C_2H_5)_2$ | 2 | H | |
| 16 | $4-F-C_6H_4-$ | 5 | $-N(CH_3)-(CH_2)_2-N(CH_3)\ C_2H_5$ | 2 | H | |
| 17 | 3-Thienyl | 5 | $-OCH_2-CH_2-N(C_2H_5)_2$ | 2 | H | |
| 18 | $3-F-C_6H_4-$ | 5 | $-OCH_2-CH_2-N(C_2H_5)_2$ | 2 | H | |
| 19 | 4-Pyridyl- | 5 | $-OCH_2-CH_2-N(C_2H_5)_2$ | 2 | H | |
| 20 | $4-OCH_3-C_6H_4-$ | 5 | $-SCH_2-CH_2-N(C_2H_5)_2$ | 2 | H | |
| 21 | 2-Thienyl- | 5 | $-SCH(CH_3)-CH_2-N(CH_3)_2$ | 2 | H | |
| 22 | $4-F-C_6H_4-$ | 5 | $-SCH_2-CH_2-N(C_2H_5)_2$ | 2 | H | |
| 23 | $4-OH-C_6H_4-$ | 5 | $-OCH_2-CH_2-N(C_2H_5)_2$ | 2 | H | |

Tabelle 2:

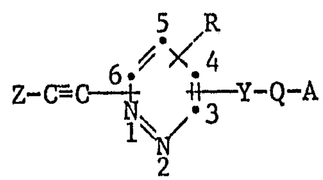

| Nr. | Z- | Stellung von $-C\equiv C-Z$ | -Y-Q-A | Stellung von -Y-Q-A | R | phys. Daten |
|---|---|---|---|---|---|---|
| 101 | $C_6H_5-$ | 6 | $-OCH_2-CH_2-N(C_2H_5)_2$ | 3 | H | Smp.69-71°C |
| 102 | $C_6H_5-$ | 6 | $-OCH_2-CH_2-N(CH_3)_2$ | 4 | H | |
| 103 | $C_6H_5-$ | 6 | $-OCH(CH_3)-CH_2-N(CH_3)_2$ | 3 | H | |
| 104 | $4-F-C_6H_4-$ | 6 | $-OCH_2-CH(CH_3)-N(CH_3)_2$ | 3 | H | |
| 105 | $4-OCH_3-C_6H_4-$ | 6 | $-O-(CH_2)_3-N(C_2H_5)_2$ | 3 | H | |
| 106 | 2-Thienyl- | 6 | $-OCH_2-CH_2-N(CH_3)C_2H_5$ | 4 | H | |
| 107 | $C_6H_5-$ | 6 | $-N(C_3H_7)-CH_2-CH_2-N(CH_3)_2$ | 3 | H | |
| 108 | $3-OCH_3-5-OCH_3-C_6H_3-$ | 6 | $-SCH_2-CH_2-N(CH_3)_2$ | 4 | H | |
| 109 | $C_6H_5-$ | 6 | $-OCH_2-CH_2-N(C_2H_5)_2$ | 4 | 4-Cl | |

Tabelle 3:

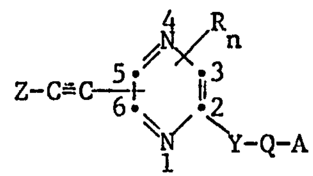

| Nr. | Z- | Stellung von -C≡C-Z | -Y-Q-A | $R_n$ |
|---|---|---|---|---|
| 151 | $C_6H_5$- | 6 | $-OCH_2-CH_2-N(C_2H_5)_2$ | H |
| 152 | $C_6H_5$- | 5 | $-OCH_2-CH_2-N(C_2H_5)_2$ | H |
| 153 | $C_6H_5$- | 6 | $-OCH_2-CH_2-N(C_2H_5)_2$ | 3-Cl,5-Cl |
| 154 | $C_6H_5$- | 5 | $-OCH_2-CH_2-N(C_2H_5)_2$ | 3-Cl,6-Cl |
| 155 | $4-F-C_6H_4-$ | 6 | $-OCH_2-CH_2-N(CH_3)C_3H_7$ | H |
| 156 | $4-OCH_3-C_6H_4-$ | 5 | $-OCH(CH_3)-CH_2-N(CH_3)_2$ | H |
| 157 | 2-Thienyl- | 5 | $-O-(CH_2)_3-N(CH_3)_2$ | H |
| 158 | 2-Thienyl- | 5 | $-SCH_2-CH_2-N(CH_3)_2$ | H |
| 159 | 2-Cl-5-Pyridyl- | 5 | $-N(CH_3)-CH_2-CH_2-N(CH_3)_2$ | H |
| 160 | 3-Thienyl | 5 | $-OCH_2-CH_2-N(C_2H_5)_2$ | H |

Tabelle 4:

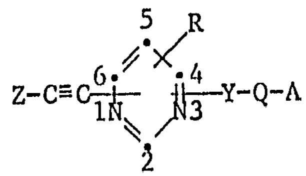

| Nr. | Z- | Stellung von $-C{\equiv}C-Z$ | -Y-Q-A | Stellung von -Y-Q-A | R |
|---|---|---|---|---|---|
| 201 | $C_6H_5-$ | 2 | $-OCH_2-CH_2-N(C_2H_5)_2$ | 5 | H |
| 202 | $C_6H_5-$ | 2 | $-OCH(CH_3)-CH_2-N(CH_3)_2$ | 5 | H |
| 203 | $C_6H_5-$ | 2 | $-OCH_2-CH(CH_3)-N(CH_3)_2$ | 5 | H |
| 204 | $C_6H_5-$ | 2 | $-O-(CH_2)_3-N(C_2H_5)_2$ | 5 | H |
| 205 | $C_6H_5-$ | 2 | $-OCH_2-CH_2-N(CH_3)C_2H_5$ | 5 | H |
| 206 | 2-Thienyl- | 2 | $-OCH_2-CH_2-N(C_2H_5)_2$ | 5 | H |
| 207 | 2-Thienyl- | 2 | $-OCH(CH_3)-CH_2-N(CH_3)_2$ | 5 | H |
| 208 | 2-Thienyl- | 2 | $-OCH_2-CH(CH_3)-N(CH_3)_2$ | 5 | H |
| 209 | 2-Thienyl- | 2 | $-OCH_2-CH_2-N(CH_3)C_2H_5$ | 5 | H |
| 210 | $4-F-C_6H_4-$ | 2 | $-OCH_2-CH_2-N(C_2H_5)_2$ | 5 | H |
| 211 | $4-F-C_6H_4-$ | 2 | $-OCH(CH_3)-CH_2-N(CH_3)_2$ | 5 | H |
| 212 | $4-F-C_6H_4-$ | 2 | $-OCH_2-CH(CH_3)-N(CH_3)_2$ | 5 | H |
| 213 | $4-F-C_6H_4-$ | 2 | $-OCH_2-CH_2-N(CH_3)C_2H_5$ | 5 | H |
| 214 | $4-OCH_3-C_6H_4-$ | 2 | $-OCH_2-CH_2-N(C_2H_5)_2$ | 5 | H |
| 215 | $4-OCH_3-C_6H_4-$ | 2 | $-OCH(CH_3)-CH_2-N(CH_3)_2$ | 5 | H |
| 216 | $4-OCH_3-C_6H_4-$ | 2 | $-OCH_2-CH(CH_3)-N(CH_3)_2$ | 5 | H |

Tabelle 4: (Fortsetzung)

| Nr. | Z- | Stellung von -C≡C-Z | -Y-Q-A | Stellung von -Y-Q-A | R | phys. Daten |
|---|---|---|---|---|---|---|
| 217 | 4-$OCH_3$-$C_6H_4$- | 2 | $-OCH_2-CH_2-N(CH_3)C_2H_5$ | 5 | H | |
| 218 | $C_6H_5$- | 5 | $-OCH_2-CH_2-N(C_2H_5)_2$ | 2 | H | Oel |
| 219 | $C_6H_5$- | 5 | $-OCH(CH_3)-CH_2-N(CH_3)_2$ | 2 | H | |
| 220 | $C_6H_5$- | 5 | $-OCH_2-CH(CH_3)-N(CH_3)_2$ | 2 | H | |
| 221 | $C_6H_5$- | 5 | $-OCH_2-CH_2-N(CH_3)C_2H_5$ | 2 | H | |
| 222 | $C_6H_5$- | 5 | $-O-(CH_2)_3-N(C_2H_5)_2$ | 2 | H | |
| 223 | 2-Thienyl- | 5 | $-OCH_2-CH_2-N(C_2H_5)_2$ | 2 | H | Smp.36°C |
| 224 | 2-Thienyl- | 5 | $-OCH(CH_3)-CH_2-N(CH_3)_2$ | 2 | H | |
| 225 | 2-Thienyl- | 5 | $-OCH_2-CH(CH_3)-N(CH_3)_2$ | 2 | H | |
| 226 | 2-Thienyl- | 5 | $-OCH_2-CH_2-N(CH_3)\ C_2H_5$ | 2 | H | |
| 227 | 4-F-$C_2H_4$- | 5 | $-OCH_2-CH_2-N(C_2H_5)_2$ | 2 | H | Smp.79-82°C |
| 228 | 4-F-$C_6H_4$- | 5 | $-OCH(CH_3)-CH_2-N(CH_3)_2$ | 2 | H | |
| 229 | 4-F-$C_6H_4$- | 5 | $-OCH_2-CH(CH_3-N(CH_3)_2$ | 2 | H | |
| 230 | 4-F-$C_6H_4$- | 5 | $-OCH_2-CH_2-N(CH_3)C_2H_5$ | 2 | H | |
| 231 | 4-$OCH_3$-$C_6H_4$- | 5 | $-OCH_2-CH_2-N(C_2H_5)_2$ | 2 | H | |
| 232 | 4-$OCH_3$-$C_6H_4$- | 5 | $-OCH(CH_3)-CH_2-N(CH_3)_2$ | 2 | H | |
| 233 | 4-$OCH_3$-$C_6H_4$- | 5 | $-OCH_2-CH(CH_3)-N(CH_3)_2$ | 2 | H | |
| 234 | 4-$OCH_3$-$C_6H_4$- | 5 | $-OCH_2-CH_2-N(CH_3)C_2H_5$ | 2 | H | |
| 235 | $C_6H_5$- | 2 | $-OCH_2-CH_2-N(C_2H_5)_2$ | 4 | H | |

Tabelle 4: (Fortsetzung)

| Nr. | Z- | Stellung von $-C{\equiv}C-Z$ | -Y-Q-A | Stellung von -Y-Q-A | R |
|---|---|---|---|---|---|
| 236 | $C_6H_5-$ | 2 | $-OCH_2-CH_2-N(C_2H_5)_2$ | 4 | $6-CH_3$ |
| 237 | $C_6H_5-$ | 2 | $-OCH_2-CH_2-N(C_2H_5)_2$ | 4 | 6-F |
| 238 | $C_6H_5-$ | 2 | $-OCH_2-CH_2-N(C_2H_5)_2$ | 4 | 6-Cl |
| 239 | $C_6H_5-$ | 2 | $-OCH(CH_3)-CH_2-N(CH_3)_2$ | 4 | H |
| 240 | $C_6H_5-$ | 2 | $-OCH(CH_3)-CH_2-N(CH_3)_2$ | 4 | 6-F |
| 241 | $C_6H_5-$ | 2 | $-OCH(CH_3)-CH_2-N(CH_3)_2$ | 4 | 6-Cl |
| 242 | $C_6H_5-$ | 2 | $-OCH_2-CH_2-N(CH_3)C_2H_5$ | 4 | 6-F |
| 243 | $C_6H_5-$ | 2 | $-OCH_2-CH(CH_3)-N(CH_3)_2$ | 4 | H |
| 244 | $C_6H_5-$ | 2 | $-OCH_2-CH(CH_3)-N(CH_3)_2$ | 4 | $6-CH_3$ |
| 245 | $C_6H_5-$ | 2 | $-OCH_2-CH(CH_3)-N(CH_3)_2$ | 4 | 6-Cl |
| 246 | $C_6H_5-$ | 2 | $-OCH_2-CH_2-N(CH_3)C_2H_5$ | 4 | H |
| 247 | $C_6H_5-$ | 2 | $-O-(CH_2)_3-N(C_2H_5)_2$ | 4 | H |
| 248 | 2-Thienyl- | 2 | $-OCH_2-CH_2-N(C_2H_5)_2$ | 4 | H |
| 249 | 2-Thienyl- | 2 | $-OCH_2-CH_2-N(C_2H_5)_2$ | 4 | $6-CH_3$ |
| 250 | 2-Thienyl- | 2 | $-OCH_2-CH_2-N(C_2H_5)_2$ | 4 | 6-Cl |
| 251 | 2-Thienyl- | 2 | $-OCH_2-CH_2-N(C_2H_5)_2$ | 4 | 6-F |
| 252 | 2-Thienyl- | 2 | $-OCH(CH_3)-CH_2-N(CH_3)_2$ | 4 | H |
| 253 | 2-Thienyl- | 2 | $-OCH(CH_3)-CH_2-N(CH_3)_2$ | 4 | $6-CH_3$ |
| 254 | 2-Thienyl- | 2 | $-OCH(CH_3)-CH_2-N(CH_3)_2$ | 4 | 6-Cl |

Tabelle 4: (Fortsetzung)

| Nr. | Z- | Stellung von $-C{\equiv}C-Z$ | -Y-Q-A | Stellung von -Y-Q-A | R |
|---|---|---|---|---|---|
| 255 | 2-Thienyl- | 2 | $-OCH_2-CH(CH_3)-N(CH_3)_2$ | 4 | H |
| 256 | 2-Thienyl- | 2 | $-OCH_2-CH(CH_3)-N(CH_3)_2$ | 4 | 6-F |
| 257 | 2-Thienyl- | 2 | $-OCH_2-CH(CH_3)-N(CH_3)_2$ | 4 | 6-Cl |
| 258 | 2-Thienyl- | 2 | $-OCH_2-CH_2-N(CH_3)C_2H_5$ | 4 | H |
| 259 | $4-F-C_6H_4-$ | 2 | $-OCH_2-CH_2-N(C_2H_5)_2$ | 4 | H |
| 260 | $4-F-C_6H_4-$ | 2 | $-OCH(CH_3)-CH_2-N(CH_3)_2$ | 4 | H |
| 261 | $4-F-C_6H_4-$ | 2 | $-OCH_2-CH(CH_3)-N(CH_3)_2$ | 4 | H |
| 262 | $4-OCH_3-C_6H_4-$ | 2 | $-OCH_2-CH_2-N(C_2H_5)_2$ | 4 | H |
| 263 | $4-OCH_3-C_6H_4-$ | 2 | $-OCH(CH_3)-CH_2-N(CH_3)_2$ | 4 | H |
| 264 | $4-OCH_3-C_6H_4-$ | 2 | $-OCH(CH_3)-CH_2-N(CH_3)_2$ | 4 | 6-F |
| 265 | $4-OCH_3-C_6H_4-$ | 2 | $-OCH_2-CH(CH_3)-N(CH_3)_2$ | 4 | H |
| 266 | 3-Thienyl- | 2 | $-OCH_2-CH_2-N(C_2H_5)_2$ | 4 | H |
| 267 | 3-Thienyl- | 2 | $-OCH_2-CH_2-N(C_2H_5)_2$ | 4 | 6-F |
| 268 | 3-Thienyl- | 2 | $-OCH(CH_3)-CH_2-N(CH_3)_2$ | 4 | H |
| 269 | 3-Thienyl- | 2 | $-O-(CH_2)_3-N(C_2H_5)_2$ | 4 | H |
| 270 | 3-Thienyl- | 2 | $-OCH_2-CH(CH_3)-N(CH_3)_2$ | 4 | H |
| 271 | 3-Thienyl- | 2 | $-OCH_2-CH_2-N(C_2H_5)_2$ | 4 | $6-CH_3$ |
| 272 | 3-Thienyl- | 2 | $-OCH_2-CH_2-N(C_2H_5)_2$ | 4 | 6-Cl |
| 273 | 3-Thienyl- | 2 | $-OCH_2-CH_2-N(C_2H_5)_2$ | 5 | H |
| 274 | 3-Thienyl- | 2 | $-OCH(CH_3)-CH_2-N(CH_3)_2$ | 5 | H |

Tabelle 4: (Fortsetzung)

| Nr. | Z- | Stellung von -C≡C-Z | -Y-Q-A | Stellung von -Y-Q-A | R | phys. Daten |
|---|---|---|---|---|---|---|
| 275 | 3-Thienyl- | 2 | $-OCH_2-CH(CH_3)-N(CH_3)_2$ | 5 | H | |
| 276 | 3-Thienyl- | 2 | $-O-(CH_2)_3-N(C_2H_5)_2$ | 5 | H | |
| 277 | 3-Thienyl- | 2 | $-OCH_2-CH_2-N(CH_3)C_2H_5$ | 5 | H | |
| 278 | 3-Thienyl- | 5 | $-OCH_2-CH_2-N(C_2H_5)_2$ | 2 | H | |
| 279 | 3-Thienyl- | 5 | $-OCH(CH_3)-CH_2-N(CH_3)_2$ | 2 | H | |
| 280 | 3-Thienyl- | 5 | $-OCH_2-CH(CH_3)-N(CH_3)_2$ | 2 | H | |
| 281 | 3-Thienyl- | 5 | $-O-(CH_2)_3-N(C_2H_5)_2$ | 2 | H | |
| 282 | 3-Thienyl- | 5 | $-OCH_2-CH_2-N(CH_3)C_2H_5$ | 2 | H | |
| 283 | $C_6H_5-$ | 4 | $-OCH_2-CH_2-N(C_2H_5)_2$ | 2 | H | Oel |
| 284 | $C_6H_5-$ | 4 | $-OCH_2-CH_2-N(C_2H_5)_2$ | 2 | $6-CH_3$ | |
| 285 | $C_6H_5-$ | 4 | $-OCH_2-CH_2-N(C_2H_5)_2$ | 2 | 6-Cl | $n_D^{25}$ 1,5940 |
| 286 | $C_6H_5-$ | 4 | $-OCH_2-CH_2-N(C_2H_5)_2$ | 2 | 6-F | |
| 287 | $C_6H_5-$ | 4 | $-OCH(CH_3)-CH_2-N(CH_3)_2$ | 2 | H | |
| 288 | $C_6H_5-$ | 4 | $-OCH(CH_3)-CH_2-N(CH_3)_2$ | 2 | $6-CH_3$ | |
| 289 | $C_6H_5-$ | 4 | $-OCH(CH_3)-CH_2-N(CH_3)_2$ | 2 | 6-Cl | |
| 290 | $C_6H_5-$ | 4 | $-OCH(CH_3)-CH_2-N(CH_3)_2$ | 2 | 6-F | |
| 291 | $C_6H_5-$ | 4 | $-OCH_2-CH(CH_3)-N(CH_3)_2$ | 2 | H | |
| 292 | $C_6H_5-$ | 4 | $-OCH_2-CH(CH_3)-N(CH_3)_2$ | 2 | $6-CH_3$ | |
| 293 | $C_6H_5-$ | 4 | $-OCH_2-CH(CH_3)-N(CH_3)_2$ | 2 | 6-Cl | |
| 294 | $C_6H_5-$ | 4 | $-OCH_2-CH(CH_3)-N(CH_3)_2$ | 2 | 6-F | |

Tabelle 4: (Fortsetzung)

| Nr. | Z- | Stellung von $-C\equiv C-Z$ | -Y-Q-A | Stellung von -Y-Q-A | R |
|---|---|---|---|---|---|
| 295 | $C_6H_5-$ | 4 | $-O-(CH_2)_3-N(C_2H_5)_2$ | 2 | H |
| 296 | $C_6H_5-$ | 4 | $-OCH_2-CH_2-N(CH_3)C_2H_5$ | 2 | H |
| 297 | 2-Thienyl- | 4 | $-OCH_2-CH_2-N(C_2H_5)_2$ | 2 | H |
| 298 | 2-Thienyl- | 4 | $-OCH_2-CH_2-N(C_2H_5)_2$ | 2 | $6-CH_3$ |
| 299 | 2-Thienyl- | 4 | $-OCH_2-CH_2-N(C_2H_5)_2$ | 2 | 6-Cl |
| 300 | 2-Thienyl- | 4 | $-OCH_2-CH_2-N(C_2H_5)_2$ | 2 | 6-F |
| 301 | 2-Thienyl- | 4 | $-OCH(CH_3)-CH_2-N(CH_3)_2$ | 2 | H |
| 302 | 2-Thienyl- | 4 | $-OCH(CH_3)-CH_2-N(CH_3)_2$ | 2 | 6-Cl |
| 303 | 2-Thienyl- | 4 | $-OCH(CH_3)-CH_2-N(CH_3)_2$ | 2 | 6-F |
| 304 | 2-Thienyl- | 4 | $-OCH(CH_3)-CH_2-N(CH_3)_2$ | 2 | $6-CH_3$ |
| 305 | 2-Thienyl- | 4 | $-OCH_2-CH(CH_3)-N(CH_3)_2$ | 2 | $6-CH_3$ |
| 306 | 2-Thienyl- | 4 | $-OCH_2-CH(CH_3)-N(CH_3)_2$ | 2 | $6-CH_3$ |
| 307 | 2-Thienyl- | 4 | $-OCH_2-CH(CH_3)-N(CH_3)_2$ | 2 | 6-Cl |
| 308 | 2-Thienyl- | 4 | $-OCH_2-CH(CH_3)-N(CH_3)_2$ | 2 | 6-F |
| 309 | 2-Thienyl- | 4 | $-O-(CH_2)_3-N(C_2H_5)_2$ | 2 | H |
| 310 | 2-Thienyl- | 4 | $-O-(CH_2)_3-N(CH_3)_2$ | 2 | H |
| 311 | 2-Thienyl- | 4 | $-OCH_2-CH_2-N(CH_3)C_2H_5$ | 2 | H |
| 312 | $4-F-C_6H_4-$ | 4 | $-OCH_2-CH_2-N(C_2H_5)_2$ | 2 | H |
| 313 | $4-F-C_6H_4-$ | 4 | $-OCH_2-CH_2-N(C_2H_5)_2$ | 2 | $6-CH_3$ |
| 314 | $4-F-C_6H_4$ | 4 | $-OCH_2-CH_2-N(C_2H_5)_2$ | 2 | 6-Cl |

Tabelle 4: (Fortsetzung)

| Nr. | Z- | Stellung von $-C\equiv C-Z$ | -Y-Q-A | Stellung von -Y-Q-A | R |
|---|---|---|---|---|---|
| 315 | $4-F-C_6H_4-$ | 4 | $-OCH_2-CH_2-N(C_2H_5)_2$ | 2 | 6-F |
| 316 | $4-F-C_6H_4-$ | 4 | $-OCH(CH_3)-CH_2-N(CH_3)_2$ | 2 | H |
| 317 | $4-F-C_6H_4-$ | 4 | $-OCH(CH_3)-CH_2-N(CH_3)_2$ | 2 | $6-CH_3$ |
| 318 | $4-F-C_6H_4-$ | 4 | $-OCH(CH_3)-CH_2-N(CH_3)_2$ | 2 | 6-Cl |
| 319 | $4-F-C_6H_4-$ | 4 | $-OCH(CH_3)-CH_2-N(CH_3)_2$ | 2 | 6-F |
| 320 | $4-F-C_6H_4-$ | 4 | $-OCH_2-CH(CH_3)-N(CH_3)_2$ | 2 | H |
| 321 | $4-F-C_6H_4-$ | 4 | $-OCH_2-CH(CH_3)-N(CH_3)_2$ | 2 | $6-CH_3$ |
| 322 | $4-F-C_6H_4-$ | 4 | $-OCH_2-CH(CH_3)-N(CH_3)_2$ | 2 | 6-F |
| 323 | $4-F-C_6H_4-$ | 4 | $-O-(CH_2)_3-N(C_2H_5)_2$ | 2 | H |
| 324 | $4-F-C_6H_4-$ | 4 | $-O-(CH_2)_3-N(CH_3)_2$ | 2 | H |
| 325 | $4-F-C_6H_4-$ | 4 | $-OCH_2-CH_2-N(CH_3)C_2H_5$ | 2 | H |
| 326 | $4-OCH_3-C_6H_4-$ | 4 | $-OCH_2-CH_2-N(C_2H_5)_2$ | 2 | H |
| 327 | $4-OCH_3-C_6H_4-$ | 4 | $-OCH_2-CH_2-N(C_2H_5)_2$ | 2 | $6-CH_3$ |
| 328 | $4-OCH_3-C_6H_4-$ | 4 | $-OCH_2-CH_2-N(C_2H_5)_2$ | 2 | 6-Cl |
| 329 | $4-OCH_3-C_6H_4-$ | 4 | $-OCH_2-CH_2-N(C_2H_5)_2$ | 2 | 6F |
| 330 | $4-OCH_3-C_6H_4-$ | 4 | $-OCH(CH_3)-CH_2-N(CH_3)_2$ | 2 | H |
| 331 | $4-OCH_3-C_6H_4-$ | 4 | $-OCH(CH_3)-CH_2-N(CH_3)_2$ | 2 | $6-CH_3$ |
| 332 | $4-OCH_3-C_6H_4-$ | 4 | $-OCH(CH_3)-CH_2-N(CH_3)_2$ | 2 | 6-Cl |
| 333 | $4-OCH_3-C_6H_4-$ | 4 | $-OCH(CH_3)-CH_2-N(CH_3)_2$ | 2 | 6-F |
| 334 | $4-OCH_3-C_6H_4-$ | 4 | $-OCH_2-CH(CH_3)-N(CH_3)_2$ | 2 | H |

Tabelle 4: (Fortsetzung)

| Nr. | Z- | Stellung von $-C\equiv C-Z$ | -Y-Q-A | Stellung von -Y-Q-A | R |
|---|---|---|---|---|---|
| 335 | $4-OCH_3-C_6H_4-$ | 4 | $-OCH_2-CH(CH_3)-N(CH_3)_2$ | 2 | $6-CH_3$ |
| 336 | $4-OCH_3-C_6H_4-$ | 4 | $-OCH_2-CH(CH_3)-N(CH_3)_2$ | 2 | 6-Cl |
| 337 | $4-OCH_3-C_6H_4-$ | 4 | $-OCH_2-CH(CH_3)-N(CH_3)_2$ | 2 | 6-F |
| 338 | $4-OCH_3-C_6H_4-$ | 4 | $-O-(CH_2)_3-N(C_2H_5)_2$ | 2 | H |
| 339 | $4-OCH_3-C_6H_4-$ | 4 | $-O-(CH_2)_3-N(CH_3)_2$ | 2 | H |
| 340 | $4-OCH_3-C_6H_4-$ | 4 | $-OCH_2-CH_2-N(CH_3)C_2H_5$ | 2 | H |
| 341 | $C_6H_5-$ | 4 | $-OCH_2-CH_2-N(C_2H_5)_2$ | 6 | H |
| 342 | $C_6H_5-$ | 4 | $-OCH(CH_3)-CH_2-N(CH_3)_2$ | 6 | H |
| 343 | $C_6H_5-$ | 4 | $-OCH_2-CH(CH_3)-N(CH_3)_2$ | 6 | H |
| 344 | $C_6H_5-$ | 4 | $-O-(CH_2)_3-N(C_2H_5)_2$ | 6 | H |
| 345 | $C_6H_5-$ | 4 | $-O-(CH_2)_3-N(CH_3)_2$ | 6 | H |
| 346 | 2-Thienyl- | 4 | $-OCH_2-CH_2-N(C_2H_5)_2$ | 6 | H |
| 347 | 2-Thienyl- | 4 | $-OCH(CH_3)-CH_2-N(CH_3)_2$ | 6 | H |
| 348 | 2-Thienyl- | 4 | $-OCH_2-CH(CH_3)-N(CH_3)_2$ | 6 | H |
| 349 | 3-Thienyl- | 4 | $-OCH_2-CH_2-N(C_2H_5)_2$ | 6 | H |
| 350 | 3-Thienyl- | 4 | $-OCH(CH_3)-CH_2-N(CH_3)_2$ | 6 | H |
| 351 | 3-Thienyl- | 4 | $-OCH_2-CH(CH_3)-N(CH_3)_2$ | 6 | H |
| 352 | $4-F-C_6H_4-$ | 4 | $-OCH_2-CH_2-N(C_2H_5)_2$ | 6 | H |
| 353 | $4-F-C_6H_4-$ | 4 | $-OCH(CH_3)-CH_2-N(CH_3)_2$ | 6 | H |

Tabelle 4: (Fortsetzung)

| Nr. | Z- | Stellung von $-C\equiv C-Z$ | -Y-Q-A | Stellung von -Y-Q-A | R |
|---|---|---|---|---|---|
| 354 | $4-F-C_6H_4-$ | 4 | $-OCH_2-CH(CH_3)-N(CH_3)_2$ | 6 | H |
| 355 | $4-OCH_3-C_6H_4-$ | 4 | $-OCH_2-CH_2-N(C_2H_5)_2$ | 6 | H |
| 356 | $4-OCH_3-C_6H_4-$ | 4 | $-OCH(CH_3)-CH_2-N(CH_3)_2$ | 6 | H |
| 357 | $4-OCH_3-C_6H_4-$ | 4 | $-OCH_2-CH(CH_3)-N(CH_3)_2$ | 6 | H |
| 358 | 4-Pyridyl- | 5 | $-OCH_2-CH_2-N(C_2H_5)_2$ | 2 | H |
| 359 | $4-CH_3-C_6H_4-$ | 5 | $-OCH_2-CH_2-N(C_2H_5)_2$ | 2 | H |
| 360 | $3-F-C_6H_4-$ | 5 | $-OCH_2-CH_2-N(C_2H_5)_2$ | 2 | H |
| 361 | $3-F-C_6H_4-$ | 5 | $-OCH(CH_3)-CH_2-N(CH_3)_2$ | 2 | H |
| 362 | $3-F-C_6H_4-$ | 5 | $-OCH_2-CH(CH_3)-N(CH_3)_2$ | 2 | H |
| 363 | $3-OCH_3-C_6H_4-$ | 5 | $-OCH_2-CH_2-N(C_2H_5)_2$ | 2 | H |
| 364 | $3-OCH_3-C_6H_4-$ | 5 | $-OCH(CH_3)-CH_2-N(CH_3)_2$ | 2 | H |
| 365 | $3-OCH_3-C_6H_4-$ | 5 | $-OCH_2-CH(CH_3)-N(CH_3)_2$ | 2 | H |
| 366 | $4-OCF_3-C_6H_4-$ | 5 | $-OCH_2-CH_2-N(C_2H_5)_2$ | 2 | H |
| 367 | $4-OH-C_6H_4-$ | 5 | $-OCH_2-CH_2-N(C_2H_5)_2$ | 2 | H |
| 368 | $4-Cl-C_6H_4-$ | 5 | $-OCH_2-CH_2-N(C_2H_5)_2$ | 2 | H |
| 369 | $4-NO_2-C_6H_4-$ | 5 | $-OCH_2-CH_2-N(C_2H_5)_2$ | 2 | H |
| 370 | $4-CF_3-C_6H_4-$ | 5 | $-OCH_2-CH_2-N(C_2H_5)_2$ | 2 | H |

Tabelle 5:

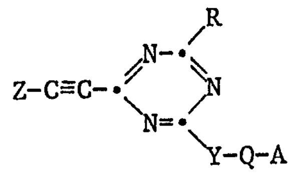

| Nr. | Z | -Y-Q-A | R |
|---|---|---|---|
| 401 | $C_6H_5-$ | $-OCH_2-CH_2-N(C_2H_5)_2$ | H |
| 402 | $C_6H_5-$ | $-OCH_2-CH_2-N(C_2H_5)_2$ | Cl |
| 403 | $C_6H_5-$ | $-OCH_2-CH_2-N(C_2H_5)_2$ | $-NH-i-C_3H_7$ |
| 404 | $C_6H_5-$ | $-OCH_2-CH_2-N(C_2H_5)_2$ | $OCH_3$ |
| 405 | 2-Thienyl- | $-OCH(CH_3)-CH_2-N(CH_3)_2$ | Cl |
| 406 | 2-Thienyl- | $-OCH_2-CH(CH_3)-N(CH_3)_2$ | H |
| 407 | $4-F-C_6H_4-$ | $-O-(CH_2)_3-N(CH_3)C_2H_5$ | $-O-C_6H_5$ |
| 408 | $4-OCH_3-C_6H_4-$ | $-O-(CH_2)_3-$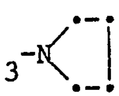 | $-N(CH_3)_2$ |
| 409 | 2-Pyridyl- | $-SCH_2-CH_2-N(CH_2-CH=CH_2)_2$ | Cl |

Tabelle 6: Z-C≡C-Het-Y-Q-A

| Nr. | Z | -Het- | -Y-Q-A |
|---|---|---|---|
| 451 | $C_6H_5$- | 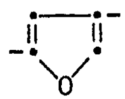 | $-OCH_2-CH_2-N(C_2H_5)_2$ |
| 452 | $4-F-C_6H_4$- | 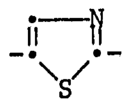 | $-OCH_2-CH_2-N(CH_3)_2$ |
| 453 | 2-Thienyl- | 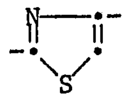 | $-OCH_2-CH_2-N(CH_3)C_2H_5$ |
| 454 | $4-OCH_3-C_6H_4$- | 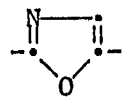 | $-OCH(CH_3)-CH_2-N(CH_3)_2$ |
| 455 | $C_6H_5$- | 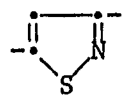 | $-OCH_2-CH_2-N$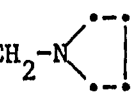 |
| 456 | $C_6H_5$- | 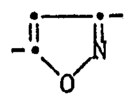 | $-OCH_2-CH_2-N(CH_3)_2$ |
| 457 | $3-OCH_3-5-OCH_3-C_6H_3$- | 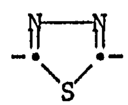 | $-O-(CH_2)_3-N(CH_3)C_3H_7$ |
| 458 | 6-Cl-3-Pyridyl- | 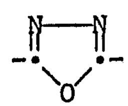 | $-OCH_2-CH(CH_3)-N(CH_3)_2$ |
| 459 | $C_6H_5$- | 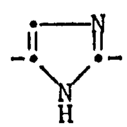 | $-OCH_2-CH_2-N(C_2H_5)_2$ |

Tabelle 6: (Fortsetzung)

| Nr. | Z | -Het- | -Y-Q-A |
|---|---|---|---|
| 460 | $C_6H_5-$ | 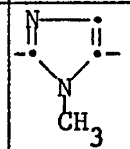 | $-OCH_2-CH_2-$ 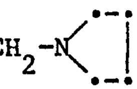 |
| 461 | $C_6H_5-$ | 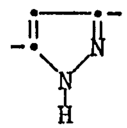 | $-OCH_2-CH_2-N(CH_3)_2$ |
| 462 | $4-CN-C_6H_4-$ | 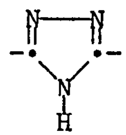 | $-OCH_2-CH_2-N(CH_3)_2$ |
| 463 | $4-F-C_6H_4-$ | 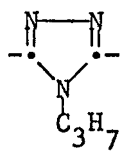 | $-OCH(CH_3)-CH_2-N(C_2H_5)_2$ |
| 464 | $C_6H_5-$ | 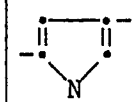 | $-OCH_2-CH_2-N(CH_3)_2$ |
| 465 | $C_6H_5-$ | 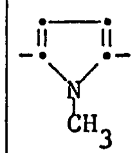 | $-O-(CH_2)_3-$ 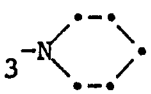 |

Tabelle 6: (Fortsetzung)

| Nr. | Z | -Het- | -Y-Q-A |
|---|---|---|---|
| 466 | $C_6H_5-$ | 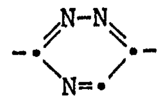 | $-OCH_2-CH_2-N(CH_3)_2$ |
| 467 | $4-F-C_6H_4-$ | 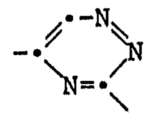 | $-OCH_2-CH_2-N(C_2H_5)_2$ |
| 468 | $C_6H_5-$ | 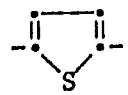 | $-OCH_2-CH_2-N(C_2H_5)_2$ |
| 469 | $4-F-C_6H_4-$ | 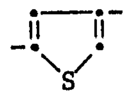 | $-OCH_2-CH_2-N(C_2H_5)_2$ |
| 470 | 2-Thienyl- | 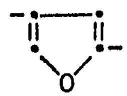 | $-OCH_2-CH_2-N(CH_3)C_2H_5$ |

Formulierungsbeispiele

Beispiel 4: Formulierungsbeispiele für flüssige Wirkstoffe der Formel I (% = Gewichtsprozent)

a) Emulsionskonzentrate

| | a) | b) | c) |
|---|---|---|---|
| Wirkstoff | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | - | - |
| Tributylphenoyl-polyäthylenglykoläther (30 Mol AeO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

b) Lösungen

| | a) | b) | c) | d) |
|---|---|---|---|---|
| Wirkstoff | 80 % | 10 % | 5 % | 95 % |
| Aethylenglykol-monomethyl-äther | 20 % | - | - | - |
| Polyäthylenglykol M G 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

c) Granulate

| | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

d) Stäubemittel

| | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Beispiel 5: Formulierungsbeispiele für feste Wirkstoffe der Formel I (% = Gewichtsprozent)

a) Spritzpulver

| | a) | b) |
|---|---|---|
| Wirkstoff | 20 % | 60 % |
| Na-Ligninsulfonat | 5 % | 5 % |
| Na-Laurylsulfat | 3 % | - |
| Na-Diisobutylnaphthalinsulfonat | - | 6 % |
| Octylphenolpolyäthylenglykoläther 7-8 Mol AeO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zur Suspensionen jeder gewünschten Konzentration verdünnen lassen.

b) Emulsions-Konzentrat

| | |
|---|---|
| Wirkstoff | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

d) Extruder Granulat

| | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff | 3 % |
| Polyäthylenglykol | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | |
|---|---|
| Wirkstoff | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 5 |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel 6: Post-emergente Herbizid-Wirkung (Kontaktherbizid)

Sowohl monokotyle wie dikotyle Unkräuter wurden nach dem Auflaufen (in 4- bis 6-Blattstadium) mit einer wässrigen Wirkstoffdispersion in einer Dosierung von 4 kg Wirksubstanz pro Hektar bespritzt und bei 24°-26°C und 45-60% relativer Luftfeuchtigkeit gehalten. 19 Tage nach der Behandlung wird der Versuch ausgewertet und das Ergebnis nach folgender Notenskala bewertet:

1: Pflanzen total abgestorben,
2-3 : sehr starke Wirkung,
4-6 : mittlere Wirkung,
7-8 : geringe Wirkung,
9: keine Wirkung (wie unbehandelte Kontrolle).

Post-emergente Wirkung

Aufwandmenge: 4 kg Wirksubstanz/Hektar

| Verb. Nr. | Setaria | Solanum | Sinapis | Stellaria |
|---|---|---|---|---|
| 1 | 4 | 2 | 2 | 3 |
| 3 | 6 | 2 | 2 | 3 |
| 4 | 4 | 1 | 1 | 1 |
| 5 | 2 | 1 | 1 | 1 |
| 6 | 7 | 1 | 1 | 3 |

Beispiel 7: Nachweis der Selektivität im Nachauflauf. In der gleichen Versuchsanordnung werden eine grössere Anzahl Kulturpflanzen und Unkräuter mit verschiedenen Aufwandmengen pro Hektar an Wirksubstanz behandelt. Die Bewertung erfolgt nach 19 Tage nach dem gleichen Massstab wie im Beispiel 6.

Post-emergente Wirkung: Verbindung Nr. 4

| Testpflanze | Aufwandmenge kg AS/ha | | |
|---|---|---|---|
| | 2 | 1 | 0.5 |
| Weizen | 8 | 9 | 9 |
| Mais | 8 | 9 | 9 |
| Reis trocken | 9 | 9 | 9 |
| Alopecurus myos. | 2 | 3 | 8 |
| Rottboellia ex. | 6 | 8 | 9 |
| Abutilon | 1 | 1 | 1 |
| Xanthium Sp | 1 | 2 | 2 |
| Chenopodium Sp. | 2 | 2 | 3 |
| Ipomoea | 1 | 1 | 1 |
| Sinapis | 1 | 1 | 1 |
| Galium aparine | 1 | 2 | 2 |
| Viola tricolor | 3 | 3 | 3 |

Patentansprüche

1. Heterocyclische Acetylenverbindungen der Formel I

$$Z-C\equiv C-Het-Y-Q-A \qquad (I)$$

worin

Het einen gegebenenfalls durch ein bis drei Reste R substituierten 5-6-gliedrigen heterocyclischen Rest, der als Ringglieder 1 bis 3 Heteroatome aus der Gruppe Stickstoff, Sauerstoff und Schwefel enthält, wobei Sauerstoff und Schwefel höchstens einmal pro Heterocyclus vorhanden sind,

A eine Gruppe $-NR_1R_2$ oder $-N^{\oplus}HR_1R_2X^{\ominus}$,

$R_1$ und $R_2$ unabhängig voneinander jeweils Wasserstoff, $C_3$-$C_6$-Alkenyl, $C_3$-$C_6$-Alkinyl, $C_3$-$C_8$-Cycloalkyl oder gegebenenfalls durch Halogen, Hydroxy, $C_1$-$C_4$-Alkoxy, Alkoxycarbonyl mit höchstens 5 Kohlenstoffatomen, Cyan oder Carboxyl substituiertes $C_1$-$C_6$-Alkyl, oder

$R_1$ und $R_2$ zusammen mit dem sie tragenden Stickstoffatom auch einen 5- bis 6-gliedrigen gesättigten Heterocyclus mit insgesamt höchstens 2 Heteroatomen, der durch $C_1$-$C_3$-Alkyl substituiert sein kann,

$X^{\ominus}$ ein Anion,

Y Sauerstoff, Schwefel oder ein Radikal $-NR_3-$,

$R_3$ Wasserstoff, $C_3$-$C_6$-Alkenyl, $C_3$-$C_6$-Alkinyl, $C_3$-$C_8$-Cycloalkyl oder gegebenenfalls durch Hydroxy, $C_1$-$C_4$-Alkoxy, Alkoxycarbonyl mit höchstens 5 Kohlenstoffatomen, Cyan oder Carboxyl substituiertes $C_1$-$C_6$-Alkyl,

Q eine unverzweigte oder verzweigte $C_2$-$C_6$-Alkylenbrücke,

R Wasserstoff, Nitro, Cyan, Trifluormethyl, $C_1$-$C_4$-Alkoxy, $C_1$-$C_4$-Alkylthio, $-NR_4R_5$, $-CO-NR_6R_7$, $-COOR_8$, $-CO-SR_9$, Halogen, $-N_3$ oder gegebenenfalls durch $C_1$-$C_4$-Alkoxy, Hydroxy, Cyan oder $-COOR_8$ substituiertes $C_1$-$C_4$-Alkyl,

$R_4$, $R_6$, $R_7$, $R_8$ und $R_9$ unabhängig voneinander jeweils Wasserstoff oder $C_1$-$C_6$-Alkyl, $C_3$-$C_8$-Alkenyl oder $C_3$-$C_8$-Alkinyl,
$R_5$ Wasserstoff, $C_1$-$C_6$-Alkyl, $C_3$-$C_8$-Alkenyl, $C_3$-$C_8$-Alkinyl, -CO-$R_{10}$, -COO-$R_{11}$ oder -CO-NH$R_{12}$ darstellen, wobei
$R_{10}$, $R_{11}$ und $R_{12}$ die gleiche Bedeutung wie $R_3$ haben und
Z für einen gegebenenfalls durch ein bis drei Reste, die dieselbe Bedeutung haben wie für R angegeben, oder Formyl, $-SO_2-NR_6R_7$, $-NH-NH_2-$, -NHOH, $-SO-R_8$, $-SO_2-R_8$, $-N=CH-NR_6R_7$, $COO^{\ominus}M^{\oplus}$ oder gegebenenfalls durch Nitro, Cyano oder $-COOR_8$ substituiertes $C_2$-$C_6$-Alkenyl bedeuten, substituierten Phenyl-, Naphthyl- oder heterocyclischen, aromatischen Rest steht, der mindestens ein Sauerstoff-, Schwefel- oder Stickstoffatom enthält, wobei $M^{\oplus}$ für ein Natrium-, Kalium-, Calcium- oder Magnesium-Kation steht.

2. Verbindungen gemäss Anspruch 1, dadurch gekennzeichnet, dass Het einen gegebenenfalls substituierten Pyridin- , Pyrazin-, Pyridazin-, Pyrimidin- oder Triazinring bedeutet.

3. Verbindungen gemäss Anspruch 1, dadurch gekennzeichnet, dass Het einen gegebenenfalls substituierten Furan-, Thiophen-, Pyrazol-, Imidazol-, Triazol-, Oxazol-, Thiazol-, Thiadiazol- oder Oxadiazolring bedeutet.

4. Verbindungen gemäss Anspruch 1,dadurch gekennzeichnet, dass Z gegebenenfalls substituiertes Phenyl oder Thienyl bedeutet.

5. Verbindungen gemäss Anspruch 1, dadurch gekennzeichnet, dass die direkte Alkylenbrücke zwischen dem Brückenatom Y und dem Stickstoffatom der Gruppe A aus 2 oder 3 Kohlenstoffatomen besteht.

6. Verbindungen gemäss Anspruch 1, dadurch gekennzeichnet, dass die Reste $R_1$ und $R_2$ für Methyl oder Aethyl stehen.

7. Verbindungen gemäss Anspruch 1, dadurch gekennzeichnet, dass das Brückenglied Y Sauerstoff ist.

8. Verbindungen gemäss Anspruch 1, dadurch gekennzeichnet, dass Het einen gegebenenfalls substituierten Pyridin-, Pyrazin-, Pyridazin-, Pyrimidin- oder Triazinring, Z einen gegebenenfalls substituierten Phenyl- oder Thienylring, $R_1$ und $R_2$ Methyl oder Aethyl und Y Sauerstoff bedeuten und die direkte Alkylenbrücke zwischen dem Brückenatom Y und dem Stickstoffatom der Gruppe A aus 2 oder 3 Kohlenstoffatomen besteht.

9. Verbindungen gemäss Anspruch 1, dadurch gekennzeichnet, dass Het einen gegebenenfalls substituierten Furan-, Thiophen-, Imidazol-, Triazol-, Oxazol-, Thiazol, Thiadiazol- oder Oxadiazolring, Z einen gegebenenfalls substituierten Phenyl- oder Thienylring, $R_1$ und $R_2$ Methyl oder Aethyl und Y Sauerstoff bedeuten und die direkte Alkylenbrücke zwischen dem Brückenatom Y und dem Stickstoffatom der Gruppe A aus 2 oder 3 Kohlenstoffatomen besteht.

10. Verbindungen gemäss Anspruch 8, dadurch gekennzeichnet, dass Het einen gegebenenfalls substituierten Pyridin- oder Pyrimidinring und Z einen gegebenenfalls substituierten Thiophenring bedeuten.

11. Verbindungen gemäss Anspruch 8, dadurch gekennzeichnet, dass Het einen gegebenenfalls substituierten Pyridin- oder Pyrimidinring und Z einen gegebenenfalls substituierten Phenylring bedeuten.

12. Verbindungen gemäss einem der Ansprüche 10 oder 11, dadurch gekennzeichnet, dass die Alkylenreste zwischen dem Brückenatom Y und dem Stickstoffatom der Gruppe A 2 Kohlenstoffatome enthält.

13. Die Verbindung 1-(5-Phenyläthinyl-pyridyl-2-oxy)-2-diäthylamino-äthan gemäss Anspruch 1.

14. Die Verbindung 2-(5-Phenyläthinyl-pyridyl-2-oxy)-1-diäthylamino-propan gemäss Anspruch 1.

15. Die Verbindung 1-[5-(2-Thienyläthinyl)-pyridyl-2-oxy]-2-diäthyl-aminoäthan gemäss Anspruch 1.

16. Verbindungen der Formel IX

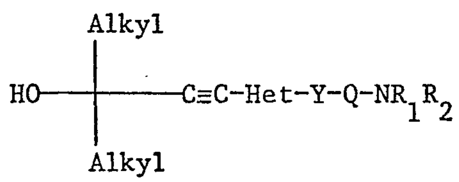

(IX)

worin Het, Y und Q die unter Formel I und $R_1$ und $R_2$ die unter Formel Ia gegebene Bedeutung haben und Alkyl eine $C_1$-$C_4$-Alkylgruppe bedeutet.

17. Verfahren zur Herstellung von Verbindungen der Formel I, gemäss Anspruch 1, dadurch gekennzeichnet, dass man entweder eine Aethinylverbindung der Formel II

$$Z - C \equiv CH \qquad (II)$$

in Gegenwart eines säurebindenden Mittels und eines Metallkatalysators vorzugsweise unter einer Inertgasatmosphäre mit einem heterocyclischen Halogenid der Formel III

$$Hal\text{-}Het\text{-}Y\text{-}Q\text{-}NR_1R_2 \qquad (III)$$

umsetzt und gegebenenfalls in die Ammoniumsalze überführt, oder dass man ein aromatisches Halogenid der Formel V

$$Z - Hal \qquad (V)$$

in Gegenwart eines säurebindenden Mittels und eines Metallkatalysators vorzugsweise unter einer Inertgasatmosphäre mit einem heterocyclischen Acetylen-Derivat der Formel IV

$$HC{\equiv}C\text{-}Het\text{-}Y\text{-}Q\text{-}NR_1R_2 \qquad \text{(VI)}$$

worin Het, Q, Y und Z die unter Formel I und $R_1$ und $R_2$ die unter Formel I gegebene Bedeutung haben und Hal Brom oder Jod ist, umsetzt und gegebenenfalls in die Ammoniumsalze überführt.

18. Verfahren zur Herstellung der Verbindungen der Formel I, gemäss Anspruch 1, dadurch gekennzeichnet, dass man ein aromatisches Halogenid der Formel V

$$Z - Hal \qquad \text{(V)}$$

in Gegenwart eines säurebindenden Mittels und eines Metallkatalysators gegebenenfalls unter einer Inertgasatmosphäre mit einem Propargylalkohol der Formel VII

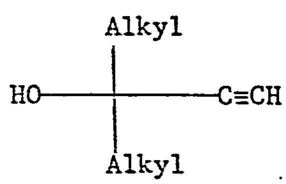

(VII)

umsetzt und die erhaltene Aethinylverbindung der Formel VIII

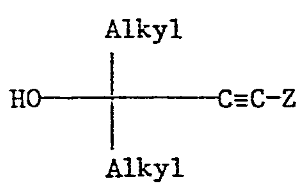

(VIII)

in Gegenwart einer starken Base und eines Metallkatalysators gegebenenfalls unter einer Inertgasatmosphäre mit einem heterocyclischen Halogenid der Formel III

$$\text{Hal-Het-Y-Q-}NR_1R_2 \qquad \text{(III)}$$

umsetzt und gegebenenfalls in die Ammoniumsalze überführt, oder dass man ein heterocyclisches Halogenid der Formel III in Gegenwart eines Metallkatalysators gegebenenfalls unter einer Inertgasatmosphäre zuerst mit dem Propargylalkohol der Formel VII umsetzt und dann die entstandene Aethinylverbindung der Formel IX

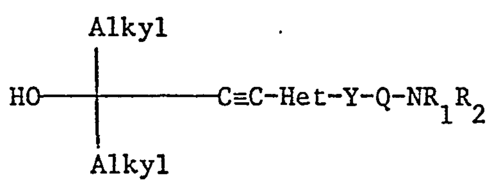

(IX)

in Gegenwart einer starken Base und eines Metallkatalysators gegebenenfalls unter einer Inertgasatmosphäre mit dem aromatischen Halogenid der Formel V, worin Het, Q, Y und Z die unter Fromel I und $R_1$ und $R_2$ die unter Formel I gegebene Bedeutung haben, Hal Brom oder Jod ist und Alkyl ein $C_1$-$C_4$-Alkylrest ist, umsetzt und gegebenenfalls in die Ammoniumsalze überführt.

19. Verfahren zur Herstellung von Ammoniumsalzen der Verbindung der Formel I, dadurch gekennzeichnet, dass man die freien Aminoverbindungen der Formel I mit einer Säure der Formel HX umsetzt und das Salz isoliert.

20. Herbizides Mittel, dadurch gekennzeichnet, dass es neben Träger- und/oder anderen Zuschlagstoffen als Wirkstoff mindestens eine heterocyclische Acetylenverbindung der Formel I, Anspruch 1, enthält.

21. Herstellung eines herbiziden Mittels gemäss Anspruch 20, dadurch gekennzeichnet, dass man Träger- und/oder andere Zuschlagstoffe mit den Wirkstoffen der Formel I innig vermischt und vermahlt.

22. Die Verwendung der heterocyclischen Acetylenverbindungen der Formel I, Anspruch 1, oder sie enthaltender Mittel zur Bekämpfung von Unkräutern.

23. Die Verwendung gemäss Anspruch 22 zur selektiven postemergenten Bekämpfung von Unkräutern in Nutzpflanzenkulturen.

24. Die Verwendung gemäss Anspruch 23 in Getreide, Mais und Reis.

25. Verfahren zur Bekämpfung von Unkräutern, dadurch gekennzeichnet, dass man eine wirksame Menge eines Wirkstoffes der Formel I auf die Pflanzen oder ihren Lebensraum appliziert.